(19) Europäisches Patentamt
European Patent Office
Office européen des brevets

(11) EP 1 704 860 B1

(12) **EUROPEAN PATENT SPECIFICATION**

(45) Date of publication and mention of the grant of the patent:
**27.10.2010 Bulletin 2010/43**

(51) Int Cl.:
***A61K 31/17*** *(2006.01)* ***A61P 1/04*** *(2006.01)*

(21) Application number: **05102423.0**

(22) Date of filing: **24.03.2005**

(54) **Benzamidine derivatives for treatment and prevention of mucositis**

Benzamidin Derivative zur Behandlung und Vorbeugung von Mucositis

Des dérivés de la benzamidine pour le traitement et la prophylaxie de la mucositis

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**27.09.2006 Bulletin 2006/39**

(73) Proprietor: **ROTTAPHARM S.P.A.**
**20122 Milano (IT)**

(72) Inventors:
- **LETARI, Ornella**
  **20131, MILANO (IT)**
- **D'AMATO, Massimo Maria**
  **20052, MONZA (Milano) (IT)**
- **ZANZOLA, Simona**
  **20132, MILANO (IT)**
- **ARTUSI, Roberto**
  **20017, RHO (Milano) (IT)**
- **ROVATI, Lucio Claudio**
  **20052, MONZA (Milano) (IT)**
- **CASELLI, Gianfranco**
  **20129, MILANO (IT)**
- **GIORDANI, Antonio**
  **27100, PAVIA (IT)**

(74) Representative: **Rambelli, Paolo et al**
**Jacobacci & Partners S.p.A.**
**Corso Emilia 8**
**10152 Torino (IT)**

(56) References cited:
**WO-A-99/45910 WO-A-02/070468**

- **ON-LINE MEDICAL DICTIONARY, [Online] XP002349560 Retrieved from the Internet: URL: http://cancerweb.ncl.ac.uk/omd/> [retrieved on 2005-10-17]**
- **WRIGHT J ET AL: "Chemotherapy-induced oral mucositis: New approaches to prevention and management" EXPERT OPINION ON DRUG SAFETY 2005 UNITED KINGDOM, vol. 4, no. 2, 2005, pages 193-200, XP009055602 ISSN: 1474-0338**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 704 860 B1

## Description

BACKGROUND OF THE INVENTION

Although significant advances have been made in the management of patients undergoing cancer chemotherapy and radiotherapy, many debilitating gastrointestinal side effects remain critical issues that have an impact on the patient management. In addition to vomiting, nausea and diarrhoea, a clinically relevant adverse event is represented by mucositis. Mucositis is the result of a complex process of interactive biologic phenomena that take place in both the epithelium and the submucosa leading to the destruction of mucosal epithelium, which results in ulcerations, mainly in the mucous membranes lining the oral and digestive tract. Mucositis results in severe pain, reduced quality of life, prolonged hospitalisation, increase risk of local and systemic infection; this is an even more serious consequence of mucositis, since the lesions can act as sites of secondary infections and as portals of entry for endogenous oral microorganisms. Therefore mucositis is a significant risk factor for life-threatening systemic infection (which can be exacerbated by the concomitant neutropenia; another side effect associated with chemotherapy) and often compromises our ability to treat the underlying cancer by delaying or truncating anticancer therapy and/or impeding recovery. High-dose chemotherapy and radiation therapy selectively affect rapidly-dividing cells, both cancerous and non-cancerous. Both normal mucosal cells and malignant cells share the characteristic of fast growing or cycling; the rapid cellular turnover displayed by mucosal lining is also common to other normal tissues such as blood cells, hair and skin that are also affected by anti-cancer therapies. Accordingly, chemotherapy and radiation therapy that are directed to interrupting cancer cell growth are also affecting fast proliferating cells in the body, such as the mucosal lining. This widely accepted explanation points out why mucositis often arises as a moderate to severe complication of antineoplastic therapy such as cancer chemotherapy and/or radiation therapy (M. Duncan, Grant G., Aliment. Pharmacol. Ther., 18 , 9 , 853-74, 2003).

The best described mucositis are the ones which occur in the mouth (oral mucositis, OM) and in the gastrointestinal (GI) tract (GI mucositis, GIM), OM is a painful condition that significantly impairs chewing and swallowing, while GIM is becoming increasingly recognised as a toxicity associated with many standard-dose chemotherapy regimen commonly used in the treatment of cancer (chemotherapy-induced mucositis is present in 40-100% of patients) and with radiotherapy addressed to any part of GI tract. The small intestine is the most concerned, but also oesophagus, stomach and large intestine can be affected.

As the mucosa of the oral cavity and of the gastrointestinal tract share a common embryological origin and development it is likely they share the same basic pathogenesis with only some differences due to specific functional components of intestinal tract. The damage to the intestine is similar to the damage that occurs in oral mucosa but it acts at a much faster rate. Similar to OM, GIM is not solely due to a direct cytotoxicity effect of radiotherapy but to a sum of direct (clonogenic and apoptotic cell death) and indirect (reactive changes) effects.

The acute toxicity in GIM could be accounted for the large part to crypt cell death, resulting in the breakdown of the mucosal barrier (Sonis ST et al., Cancer, suppl. 100, 9, 1995-2025, 2004). This pivotal effect may be the result of an effect either direct or mediated by a series of intermediate steps as the crypt cell death could be a consequence of endothelial apoptosis that, as in oral mucositis, become the primary event. As previously reported, many chemotherapeutic agents kill rapidly dividing cells, making GI tract particularly vulnerable, but differently from radiation, chemotherapy-induced mucositis have been focused mainly on the small intestine. Cytotoxic agents act at different levels of the crypt cell hierarchy, leading to crypt hypoplasia followed by regeneration. The first abnormality noted in human small intestine is an increase in apoptosis on day 1 after chemotherapy; this is followed by reductions in crypt length, villus area, and mitotic index, which reach their maximal reduction on day 3. Rebound hyperplasia follows on day 5, prior to normalisation. Although more molecular events have been elucidated in the pathogenesis of oral mucositis relative to its GI counterpart, oral cavity and the GI tract have sufficient homology to expect that mucosal barrier injury in the GI tract and in the oral mucosa share similar mechanisms (Sonis ST et al, Cancer, suppl. 100, 9, 1995-2025, 2004).
Even though mucositis represents a clinical outcome due to a complex interaction of local tissue (connective tissue, endothelium, epithelium) toxicity, induced by chemotherapy or radiation and could be seen as different pathologies, recent scientific efforts in this area highlighted how a common mechanistic scheme could be recognised for the physiological basis of mucositis.

As a matter of fact, the evolution of mucosal barrier injury can be viewed as a five-phase process: the initial phase (step 1) is characterized by the generation of Reactive Oxygen Species (ROS). This is supported by studies reporting an attenuation of mucosal injury induced by agents that block or scavenge oxygen-free radicals (Facorro G et al., Bone Marrow Transplant., 33, 8, 793-8, 2004, Sonis ST et al., Cancer, suppl. 100, 9, 1995-2025, 2004). The second phase (step 2) is characterised by a series of multiple effects driven from oxidative stress. Even though ROS can directly damage DNA (leading thus to the subsequent clonogenic cell death), the more striking effect mediated by ROS is the amplification of the damage, by stimulating a number of transcription factors (Sonis ST et al., Cell Prolif., 35, Suppl 1:93-102, 2002). Among them, nuclear factor-kB (NF-kB) has been highlighted as the key element in the genesis of mucositis (Sonis ST, Nat rev Cancer, 4, 4, 277-284, 2004). NF-kB is either activated by chemotherapy or radiotherapy

and it is able to up-regulate a large panel of genes, including those that result in the production of pro-inflammatory cytokines $TNF_{\alpha}$ , IL-1 and IL-6, all leading to apoptosis and tissue injury, and up-regulation of genes that can cause the expression of adhesion molecules, cyclooxygnase-2 and iNOS. The effect of COX-2 and iNOS products in amplifying the tissue degeneration in experimental radiation-induced mucositis has been recently described in depth (Sonis ST et al., Oral Oncol., 40, 2, 170-6, 2004; The third phase (step 3) is characterised by the amplification of signalling triggered by pro-inflammatory cytokines that can activate different pathways such as ceramide and caspase pathways, all leading to a further increase in pro-inflammatory cytokines. The fourth step (step 4) is characterised by the symptoms of mucosal barrier destruction due to tissue ulceration. During this phase there is a massive infiltration of inflammatory cells and colonisation sustained by gram-positive and gram-negative bacteria. The cell wall products from bacteria can in turn activate cell tissue infiltrate and exacerbate the inflammatory reaction. This phase is very crucial for the continuation of cancer therapy and represents a serious risk of bacteraemia and/or fungal infections.

The final phase (step 5), which occurs only in the absence of infections, represents the healing phase, that starts from extracellular matrix and leads to renewal of epithelial proliferation and differentiation. After the healing phase the oral mucosa appears normal: however the mucosal environment has been altered and the patients is at risk of future episode of mucositis during anticancer therapy.

This complex biological scenario, which shows how mucositis should be considered as the result of cumulative and interactive effects of chemotherapy and/or radiation with epithelial connective tissue, endothelium pro-inflammatory cytokines, cellular elements within the mucosa as well as concomitant infections, may explain why the treatment of mucositis has been so largely empirical and, due to the lack of a specific and effective treatment, lead to either cessation of the anticancer therapy or consist of palliative and supportive intervention (Rubenstein EB, et al., Cancer suppl., 100, 9, 2026-2046, 2004; Worthington HV et al. Cochrane Review, 3, 2004).

WO99/45910 describes a method of treating mucositis by a mixture of therapeutic agents, such as an NSAID, a MMP inhibitor, a NO inhibitor, a mast cell inhibitor and an inflammatory cytokine inhibitor. However, there is no experimental evidence of therapeutic effectiveness of these mixtures.

For OM it is widely accepted that a good oral hygiene reduces the risk. Oral care protocols are widely used with the purpose of maintaining mucosal health and integrity, to reduce the impact of the oral microbial flora and to reduce symptoms such as pain and bleeding and prevent soft tissue infections that may have systemic effects. In patients under-going haematopoietic stem cell transplantation the treatment of choice for pain control is the analgesia with morphine. Other approaches include the use of systemic analgesics and palliative mixture of agents, coating agent and topical analgesics. There is no significant evidence of the effectiveness of this mixture. In patients with head and neck cancer treated with moderate radiotherapy the preventive pharmacological protocol suggests topical use of benzydamine, due to its anti-inflammatory effects beside to its analgesic and anaesthetic properties. Even though benzydamine has been extensively studied, there are no definitive trials confirming its activity in preventing or cure radiation-induced mucositis. Also to treat chemotherapy-induced mucositis: only palliative protocols are available. For high-dose chemotherapy the protocol recommends Low-Level Laser Therapy (LLLT) in an attempt to reduce the incidence of mucositis. It has been reported that LLLT promotes wound healing and reduces pain and inflammation. However this type of intervention requires a specific equipment, often expensive, specialised training, and treatment can be time consuming. Finally, one drug is suggested to reduce esophagitis induced by combined chemotherapy and radiotherapy, i.e. amifostine, due to its reported radioprotective activity. Amifostine acts as a potent ROS scavenger, unfortunately this drug is endowed with a lot of negative features: it requires iv administration, and it has acute toxicity. FDA approved its clinical use only for reduction of renal toxicity associated with cisplatin therapy in patients bearing ovarian cancer or lung cancer. Only very recently the FDA has approved the use of the human recombinant keratinocyte growth factor (rHu-KGF; palifermin), which, by increasing epithelial stem cell proliferation, differentiation and migration ensures an increase probability of epithelial cell survival and speed up the rate of cell regeneration. Palifermin use is however restricted to the treatment of mucositis only in adult patients with haematologic malignancies undergoing myelotoxic therapy requiring haematopoietic stem cell transplant (the safety and efficacy of palifermin in the treatment of mucositis has not been established in adult patients with non-haematologic malignancies nor in children with both haematologic or non-haematologic malignancies).

Due to the lack of effective pharmacological treatments, mucositis incidence is quite high in patients undergoing chemotherapy and/or radiation therapy or total body irradiation (the latter being the routine preconditioning procedure prior to bone marrow transplant).

The incidence of oral and GI mucositis varied among therapy regimens (Sonis ST et al, Cancer, suppl. 100, 9, 1995-2025, 2004): anthracycline-based regimens were associated with 1-10%, as well as for patients bearing breast cancer or non-Hodgkin lymphomas whose regimens not included 5-FU. In contrast, chemotherapy including 5-FU was associated with more than 15 % oral mucositis and chemotherapy with CPT-11 was associated with the same rate GI mucositis. Addition of radiation to chemotherapy increased the risk to more than 30%. Oral and GI mucositis frequency and severity in patients undergoing high-dose chemotherapy combined with total body irradiation with haematopoietic stem cell transplantation can occur up to 100% of these patients and it is characterised by pain, difficulty to swallow to

a total parenteral nutrition requirement, fever, risk of infection even to fatal sepsis. Radiation therapy to head and neck was associated with an even increased incidence of oral or GI mucositis, often exceeding 50 % of patients. High frequency and severity of mucositis is also present in patients with GI or gynaecologic malignancies. Acute damage to the GI mucosa is a consequence of radiotherapy in 85-100 % of patients.

This significant incidence of mucositis in patients undergoing cancer treatment also reflects in a relevant social cost, which includes increased health care resource utilisation, due to the reduction in cure rate as a result of the reduction of the dose of the anticancer therapy, prolongation of hospitalisation for fever, narcotic usage and parenteral nutrition.

Finally, even though in a less extent, mucositis is not restricted to cancer patients only, since this disease also affects HIV patients, patients affected with non-Hodgkin's lymphoma, debilitated elderly patients.

Accordingly, there is still a remarkable need for new therapies effective in the treatment and prevention of mucositis.

## GENERAL DESCRIPTION OF THE INVENTION

The subject matter of the inventions is defined by the appended claims.

The present invention relates to the use of a compound of formula (I) for preparing a medicament or pharmaceutical compositions containing an effective amount of said compound for treatment and/or prevention of mucositis.

Compounds of formula (I) represents a selected group of the compounds previously reported in the International Patent Application WO02/070468, from our group, and claimed for treatment of inflammatory and auto-immune diseases.

The present invention concerns the discovery that a selected group of benzamidine derivatives, those of formula (I) as reported above, are particularly suitable for treatment and/or prevention of mucositis, particularly mucositis induced by chemotherapy and/or radiotherapy.

As detailed below, compounds of formula (I) are able to effectively interfere with each of the mucositis phases, as described in the background, thus providing a highly efficacious pharmacological tool for prevention and treatment of mucositis.

More in detail, as reported in the background, mucositis (either OM or GIM) share a common degenerative pathway which involves five-phases or steps. The first step is represented by the action of ROS that trigger a complex series of events that characterise the second step, where, in addition to the clonogenic cell death, the activation of nuclear factors (in particular NF-kB) leads to cytokines production along with other pro-inflammatory agents (among them $PGE_2$ the main product of COX-2). During the third step the signal triggered by cytokines is amplified, giving rise to damage propagation which ultimately leads to tissue ulceration. In the forth step mucosal barrier destruction occurs, and during this phase there is a massive bacterial colonisation and infiltration of inflammatory cells; finally during the fifth step, in the absence of infection, healing occurs.

Compounds of formula (I) display a remarkable effect in preventing ROS formation in human cells, thus acting at Step 1 by preventing the triggering process for mucositis. In addition the effect of compounds of this invention stretches to step 2, as highlighted by the potent inhibitory effect on cytokines production along with other pro-inflammatory endogenous compounds such as prostaglandins ($PGE_2$) and nitric oxide (NO). In addition, compounds of formula (I) have been found to be strongly effective in reducing the clonogenic stem cell death. Accordingly, acting at both the initiation step and the subsequent propagation step, compounds of formula (I) are suitable agents for both prevention and treatment of mucositis. Avoiding or reducing both the insult and the subsequent propagation of the pro-inflammatory stimulus, these compounds exert they activity also in step 4, by preventing and treating the concurrent damage to the basal epithelial cell and the consequent mucosal breakdown, which is crucial for bacterial colonisation, they can also indirectly prevent the infection. Finally, compounds of formula (I) have shown striking mucosal protective and wound-healing properties, thus these compounds are also able to act during the healing phase (step 5).

Accordingly, this invention concerns with a new pharmacological therapy for preventing and treating mucositis, which consists in administering to a human in need thereof a pharmaceutical acceptable formulation of an effective amount of a compound of formula (I) or its pharmaceutically acceptable salt or a solvate thereof.

The term "preventing" herein means any prophylactic action aimed at avoiding, inhibiting or restraining development of mucositis in a patient in need of this. The term "treating" includes prohibiting the disease development, stopping or reversing its progression, decreasing severity or resultant clinical symptoms of the disease, as well as any improvement in the well being of patients.

The term "mucositis" has the same meaning as described in the background, and refers to oral mucositis, gastrointestinal mucositis, uro-genital and nasal tract mucositis.

The patient treated with pharmaceutical compositions of the compounds of the invention can be a cancer patient preparing to undergo chemotherapy or radiation therapy, or a cancer patient currently undergoing chemotherapy or radiation therapy, or a patient preparing to bone marrow transplant. In addition, HIV patients, patients affected with non-Hodgkin's lymphoma, debilitated elderly patients, at risk or suffering of mucositis can be treated with methods and compositions of this invention.

DETAILED DESCRIPTION OF THE INVENTION

The new use of the invention relates to the following compounds:

**Compounds of Formula (I):**

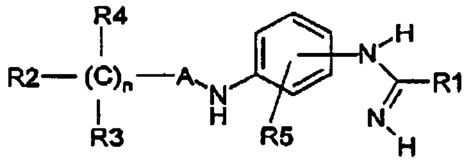

wherein:

- A is the thiocarboxamide group,
- $R_1$ is selected from an alkyl group having from 1 to 3 carbon atoms and the amino group, unsubstituted or substituted with the nitro group or the methyl group,
- $R_2$ is selected independently from hydrogen, an alkyl group having from 1 to 4 carbon atoms, a cycloalkane residue having from 5 to 7 carbon atoms, an aryl, naphtyl or heterocyclic group, unsubstituted or substituted with methyl, methoxy, hydroxy, amino or halogen groups,
- $R_3$ and $R_4$ are selected independently from hydrogen and an alkyl group having from 1 to 3 carbon atoms,
- $R_5$ represents one or two substituents independently selected from hydrogen and the methyl, methoxy and hydroxyl groups,
- n is a whole number from 0 to 6, and
- the amidine group is in the *para* or *meta* position relative to the "A-NH" group.

In the compounds of formula (I), $R_2$ is preferably linked to A through an alkylene group, having from 1 to 6 carbon atoms, optionally substituted with one or more alkyl groups having from 1 to 3 carbon atoms.
In the compound of formula (I) an aryl group is a substituted or not substituted phenyl; an heterocyclic group is a monocyclic or bicyclic aromatic heterocycle containing 1 or 2 nitrogen atoms, or a monocyclic or bicyclic aromatic heterocycle containing 1 oxygen or sulphur atom.
Non-limiting examples of heterocyclic groups are pyridine, furane, thiophene, quinoline benzofurane and benzothiophene.
The compounds of formula (I) used in the present invention can be prepared according to established procedures as described in WO02/070468, these procedures are summarised by reference herein. In general the process starts with the reaction of the appropriately substituted phenylenediamine of formula (IV)(Scheme 1) which is reacted with the corresponding isothiocyanate of formula (V a) to give rise respectively to the corresponding thiourea of formula (III a). Compounds of formula (III) are then reacted with the appropriate imidate hydrochloride of formula (II) to afford compounds of formula (I).

**Scheme 1**

Non-limiting representative examples of compounds of formula (I) of the present invention are reported below and in Table 1 :

- N-[4-(N-acetamidine)phenyl]-N'-pentyl thiourea (compound 1.1)
- 1-guanidinophenyl-4-cyclohexyl thiourea (compound 1.2)
- 1-nitroguanidinophenyl-4-cyclohexyl thiourea (compound 1.3)
- N-[4-(N-acetamidine)phenyl]-N'-butyl thiourea (compound 1.4)
- N-[4-(N-acetamidine)phenyl]-N'-(3-methyl-butyl) thiourea (compound 1.5)
- N-[4-(N-acetamidine)phenyl]-N'-[2-(4-fluorophenyl)ethyl) thiourea (compound 1.6)
- N-[4-(N-acetamidine)phenyl]-N'-[2-(4-chlorophenyl)ethyl) thiourea (compound 1.7)

Table 1:

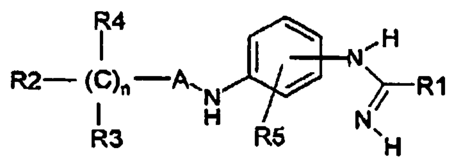

| Compound | $R_1$ | $R_2$ | $R_3$ / $R_4$ | n | A |
|---|---|---|---|---|---|
| 1.1 | $CH_3$ | $CH_3$ | H | 4 | NH-CS |
| 1.2 | $NH_2$ | cyclohexyl | - | 0 | NH-CS |
| 1.3 | $NO_2$-NH | cyclohexyl | - | 0 | NH-CS |
| 1.4 | $CH_3$ | $CH_3$ | H | 3 | NH-CS |
| 1.5 | $CH_3$ | isopropyl | H | 2 | NH-CS |
| 1.6 | $CH_3$ | 4-F-Phenyl | H | 2 | NH-CS |
| 1.7 | $CH_3$ | 4-Cl-Phenyl | H | 2 | NH-CS |

$R_5$ is always H in these compounds; the two phenyl N-H substituents are always in the *para* position.

Pharmaceutically acceptable salts of compounds of formula (I) can be particularly suitable for the preparation of pharmaceutical compositions useful for mucositis treatment, since they have enhanced water solubility compared to the compound from which they are derived. As reported below, for mucositis treatment and/or prevention in addition to the usual oral formulations such as tablets, capsules and pills, , also syrups, oral rinse, gels or emulsions can be useful formulations for this disease treatment. In addition, a considerable water solubility is essential for a proper formulation of dosage forms for parenteral administration, suitable for the treatment of the most severe forms of this disease. Finally, improved water solubility can also improve adsorption of oral formulations.

Salts of compound of formula (I) are typically formed by reacting a compound of formula (I) with an equimolar or excess amount of the appropriate acid.

Representative non limiting examples of pharmaceutically acceptable salts of compounds of formula (I) are: hydrochloride, hydrobromide, hydrogensulphate and sulphate, methansulphonate, maleate, fumarate and succinate.

In order to provide examples about the impact on solubility exerted by different pharmaceutically acceptable salts of compound of formula (I), the preparation of the maleate and of the methanesulphonate of compound 1.1 is herein reported as non limiting representative example.

N-[4-(N-acetamidine)phenyl]-N'-pentyl thiourea maleate

Compound 1.1, 1g (3.59 mmoles), is suspended in ethyl acetate (30 mL) then a solution of maleic acid, 416 mg (3.59 mmoles) in methanol (10 mL) is added on stirring at room temperature. The resulting solution is stirred at room temperature 10 minutes then concentrated in vacuum, the resulting residue is treated with a mixture of ethyl acetate (10 mL) and isopropylether (10 mL), the resulting precipitate is filtered and dried to afford 1.1 g of the maleate.
m.p. 215˚C; IR: 1681, 1622, 1543, 1511.
$^1$HNMR (DMSO-$d_6$), ppm: 0.90 (t, 3H, J= 6.2Hz); 1.31-1.36 (m, 4H); 1.53-1.59 (m, 2H); 2.32 (s, 3H); 3.37-3.48 (m, 2H); 6.06 (s, 2H); 7.25 (d, 2H, J= 8.2); 7.69 (d, 2H, J= 8.2 Hz); 7.94 (m, 1H); 8.48 (bs, 1H); 9.43 (bs, 1H); 9.73 (bs, 1H) ; 11. 1 (s, 1H); 14.3 (s, 1H).

N-[4-(N-acetamidine)phenyl]-N'-pentyl thiourea methanesulphonate

This salt is prepared from 1g of compound 1.1 and 0.23 mL (3.59 mmol) of methanesulphonic acid using the same procedure as reported above for the maleate.
IR: 1676, 1627, 1544, 1511.
$^1$HNMR (DMSO-$d_6$), ppm: 0.93 (t, 3H, J= 6.0Hz); 1.30-1.38 (m, 4H); 1.51-1.59 (m, 2H); 2.30 (s, 3H); 2.39 (s, 3H); 3.40-3.48 (m, 2H); 7.23 (d, 2H, J= 8.9); 7.69 (d, 2H, J= 8.9 Hz); 8.04 (m, 1H); 8.50 (bs, 1H); 9.43 (bs, 1H); 9.79 (bs, 1H); 11.05 (s, 1H).

The hydrochloride of compound 1.1 is prepared as reported in WO02/070468.

Solubility in water, at 25˚C, for the representative examples of salts of compound 1.1 are reported in the table

below:

| Salts for Compound 1.1* | Solubility in water (mg/mL) | Solubility in water (%) |
|---|---|---|
| Hydrochloride | 9.0 | 0.90 |
| Maleate | 3.12 | 0.31 |
| Methanesulphonate | >32 | >32 |
| (*) The compound 1.1 is not water soluble as free base. | | |

Pharmacological Activity

The compounds of the invention have been demonstrated to inhibit ROS production in human polymorphonuclear leukocyte (PMNL), to inhibit cytokine production, iNOS and COX-2 protein expression, as assessed in an "in vitro" rat model, to protect the mucosa and display wound-healing properties, as assessed in a rat model of gastric mucosa ulceration induced by indomethacin. Finally, the compounds of the invention increase crypt cell survival, as evaluated in an *"in vivo"* mucositis model in mice.

As a representative non-limiting example pharmacological data for compound 1.1 are reported below.

*Inhibition of ROS generation in human PMNL:*

Background to the assay: One of the most important events involved in the intracellular cascade leading to NF-kB activation is the generation of oxidative stress and the increase of ROS; the inhibition of these species can contribute to reduce the direct damage to DNA and subsequent clonogenic cell death, and also to diminish the transcription factors activation. The effect of compound 1.1 on luminol-dependent chemiluminescence assay was assessed in human PMNL. Data are reported in figure 1.

*Cytokine inhibition in rat peritoneal macrophages.*

Background to the assay: Nuclear factor-KB(NF-KB), a key element in the genesis of mucositis, has the capacity to up-regulate a large panel of genes, including those that result in the production of pro-inflammatory cytokines, TNFα, IL-1 and IL-6, all leading to apoptosis and tissue injury, and to up-regulate genes that can cause the expression of iNOS and cyclooxygnase-2. The third phase of mucositis is indeed characterized by the amplification of signalling triggered by pro-inflammatory cytokines. The effect of compound 1.1 was assessed in rat peritoneal macrophages. Data are reported in table 1 and 2.

**Tab.** 1: Inhibition by compound 1.1 of LPS-induced cytokine release in rat peritoneal macrophages

| | $IC_{50}$ (μM) | | |
|---|---|---|---|
| **Example** | **IL-1β** | **IL-6** | **TNFα** |
| Compound 1.1 | 30 μM | 63 μM | 54 μM |

**Tab. 2**: Inhibition by compound 1.1 of LPS-induced iNOs and COX-2 protein expression in rat macrophages

| | % of inhibition | |
|---|---|---|
| **treatment** | **iNOs** | **COX-2** |
| LPS, 0.1μg/ml, 24h | 0 | 0 |
| LPS, 0.1)μg/ml + Compound 1.1, 30μM | 79 | 53 |

*Gastric ulcer induced by indomethacin in rat: Wound-healing properties of Compound 1.1*

Indomethacin induces the formation of acute gastric mucosal lesions. The histologic damage is represented by necrosis with loss of surface epithelium, submucosal oedema and leukocyte infiltration. The mechanism involves a

neutrophil-dependent process inducing a variety of inflammatory mediators such as reactive oxygen species, and direct detrimental action by indomethacin on processes linked to epithelial proliferation and apoptosis. The epithelial repairing process is due to continuity of epithelial cells with healthy cells of gastric pits that can migrate to the basement membrane; reepithelialization and reconstruction of the mucosal architecture is under the control of growth factors produced locally by regenerating cells.

The effect of compound 1.1 was assessed in rat gastric mucosa. Data are reported in figure 2.

Finally, in vivo efficacy of the compounds of the invention was demonstrated in a mucositis model in mice.

*Mouse Mucositis model*

There are thought to be between four and sixteen actual stem cells in each crypt of the small intestine. There are also a further reserve of clonogenic cells which are capable of regenerating the crypt when all the actual stem cells have been killed. The survival of these clonogenic cells is therefore key to the survival of the crypt and the restoration of an intact epithelial lining following cytotoxic injury (only one clonogenic cell needs to survive to ensure the survival of the crypt, and therefore the maintenance of an intact epithelium). Growth factors and other molecules can be used to manipulate the sensitivity of these cells to cytotoxic agents, and thereby reduce the severity of gastrointestinal and oral mucositis. Factors given prior to a cytotoxic insult may increase clonogenic cell number (thereby increasing the probability of clonogen survival) or act to arrest the cell cycle in such cells (thereby making them more resistant to damage or death). Factors given after the insult may initiate early stem cell amplification or proliferation and hence speed up the regeneration process. A combination of both protocols could give maximum protection to the epithelium.

This study therefore examined the effectiveness of Compound 1.1 at protecting clonogenic cells, and hence crypts, from radiation induced damage. The effects of administration for 3 days before radiation exposure were tested.

The protective effect is summarized in figure 3 and detailed in table 3.

Compound 1.1 at 20mg/kg prevented the absence of surviving crypt (as seen in 4% of circumference in vehicle treated mouse), and increased the percentage of surviving crypts per circumference.

**Table 3.** Effect of Compound 1.1 on clonogenic crypt cell death induced by whole body irradiation in mouse.

| Treatment | | no. crypts/ circumference | crypt width (μm) | corrected crypts/ circumference |
|---|---|---|---|---|
| 20mg/kg Compound 1.1 for 3 days pre - 13Gy irradiation | **Average +/- sd** | **13.2 +/- 5.7** | **66.19+/-3.0** | **6.7+/-3.0** |
| 10mg/kg Compound 1.1 for 3 days pre - 13Gy irradiation | **Average +/- sd** | **9.2 +/-4.7** | **68.25+/-2.4** | **4.5+/-2.4** |
| 5mg/kg Compound 1.1 for 3 days pre - 13Gy irradiation | **Average +/- sd** | **9.6+/-3.4** | **68.54+/-6.6** | **4.7+/-1.3** |
| vehicle controls, 13Gy irradiation | **Average +/- sd** | **7.2+/-3.9** | **68.92+/-6.1** | **3.5+/- 1.9** |
| untreated controls | **Average +/- sd** | **102.8+/-5.8** | **33.66+/-1.3** | |

Pharmacological Assays

*Inhibition of chemiluminescence in human PMNL*

Human neutrophils were obtained from healthy volunteers. Blood was anti-coagulated with Na-Citrate 0.38% and neutrophils were purified according to Boyum (Boyum A. Scand J Clin Invest 1968;21:77-89). The neutrophil purification was achieved by gradient centrifugation on Histo-paque at 400 g for 30 min. Resulting neutrophils were suspended in PBS plus 0.87 mM $CaCl_2$, 1 mM $MgCl_2$, counted, and diluted to 2.5 x $10^6$ /ml. Neutrophil suspension was premixed with luminol (5 μM final). A 200 μl-aliquot of cell suspension was incubated into a 96-well plate, with drugs for 10 min at 37°C. Neutrophils were activated with 0.1 μM phorbol 12-myristate 13-acetate (PMA) and the light emission was monitored at 3 min intervals for 24 min in a HTS7000 plus microplate reader. Results were expressed as reduction of the fluorescent signal recorded for cell activated with PMA alone.

Activation of neutrophils with 0.1 μM PMA induced a time-dependent increase in signal luminescence, with a maximal increase within 10-12 min. The pre-incubation with Compound 1.1 (1-10 μM) concentration-dependently decreased luminol-enhanced chemiluminescence (rising phase, at 15 min, $IC_{50}$= 6.4±0,6 μM, steady phase, at 24 min, IC50=2,9±0,2 μM) . The inhibitory effect was detectable even at the lowest concentration of 1μM (20 % of inhibition) and at 30μM Compound 1.1 completely inhibited ROS generation (data not shown). The data are illustrated in Fig. 1.

*Cytokine inhibition in rat peritoneal macrophages*

Primary cell cultures were obtained from male albino rats (SD, 200-250g, Harlan, Italy), as described in Methods in Enzymology (Methods in Enzymology, vol. LVIII, pages 494-506). Cells were stimulated, the day after plating, with LPS, 1μg/ml or 0.1μg/ml as stated, for 24h. Compounds were added 20 min before stimulation. The stimulation was performed in DMEM, 1 g/l glucose, 50 μg/ml gentamicin. Supernatants and cell lysates were collected and stored at -80˚C until use. Cytokine quantisation in supernatants was determined by means of commercially available ELISA Kits for rat TNFα, rat IL-1β and rat IL-6 (Amersham).

Western blot analysis of iNOS and COX-2: Cell lysates were analysed by SDS-PAGE. Proteins were transferred onto PVDF membranes and saturated in blocking buffer. Membranes were incubated for 2 h at RT with the following antibodies: anti-COX-2, anti-iNOS, anti-β-actin and further incubated with a secondary antibody for 45 min at RT. Detection was performed using ECL (Amersham). Quantisation was determined by densitometry analysis using NIH Image software.

Activation of macrophages with 1 μg/ml LPS induced an increase over basal in cytokine production. The pre-incubation with Compound 1.1 (3-100 μM) concentration-dependently decreased all the three cytokines, i.e. IL-1β, IL-6 and TNFα. Compound 1.1 in the range 30-100 μM significantly inhibited cytokine production The data are illustrated in table 1.

Evaluation of mediator of inflammation such as iNOS and COX-2, was performed in macrophages stimulated with 0.1μg/ml LPS for 24h. Compound 1.1, tested at 30μM, significantly decrease both iNOs and COX-2 protein expression. The data are illustrated in table 2.

*Gastric ulcer induced by indomethacin in rat: Wound-healing properties of Compound 1.1*

20 male SD rats (140-160) were used. The animals were deprived of food but not water 24 hours prior the experiment. Gastric ulcer was induced in conscious rats by oral administration of 10 mg/kg/4ml of indomethacin, suspended in methylcellulose 0.5%. The tested drug was administered 30 min by gavage (os), or 15 min by subcutaneously (sc), before indomethacin.

Four hours after indomethacin administration, the animals were sacrificed by excess of ether. The stomach was dissected out, opened along the greater curvature, and the mucosa was examined by an observer who was unaware of the treatment given. The extent of ulcers was measured with a 10x binocular fitted with a 0.1mm-division scale. Data are presented as total length of ulcers per group.

The animals were divided into 4 groups of 5 animals, and were treated as follows:

Groups:

1. 10mg/kg Compound 1.1, administered prior to 10 mg/kg indomethacin, os.
2. 5mg/kg Compound 1.1, administered prior to 10 mg/kg indomethacin, os.
3. 1mg/kg Compound 1.1, administered prior to 10 mg/kg indomethacin, os.
4. vehicle, administered prior to 10 mg/kg indomethacin, os.

Compound 1.1 was given at 1, 5 and 10mg/kg prior to indomethacin. In vehicle treated groups all the animals exhibited ulcers. In Compound 1.1 treated groups the levels of ulcerated animals decreased dose-dependently. Maximal effect, i.e. no animals ulcerated, was achieved at the highest dose (10mg/ kg). The 5 mg/kg dose reduced about to 50% the incidence of ulcer in both administration protocol (3/5 animals), and, most important, the extent of ulceration was dramatically reduced up to 80-90%. The lower dose (1mg/kg) was effective only in the os protocol administration.

All the animals survived the treatment and exhibited no obvious adverse effects.

The data are illustrated in Figure 2.

*Mouse mucositis model*

30 adult male BDF1 mice (aged 10-12 weeks) were used. The animals were housed for 2 weeks in individually ventilated cages on a 12hr light:dark cycle to stabilise the circadian rhythm. Animals were allowed food and water ad libitum throughout.

The animals were divided into 5 groups of 6 animals, and were treated as follows:

Groups:

1. Gavage 20mg/kg Compound 1.1 72, 48 and 24hrs prior to 13Gy X-ray exposure (whole body).
2. Gavage 10mg/kg Compound 1.1 72, 48 and 24hrs prior to 13Gy X-ray exposure (whole body).
3. Gavage 5mg/kg Compound 1.1 72, 48 and 24hrs prior to 13Gy X-ray exposure (whole body).
4. Gavage vehicle 72, 48 and 24hrs prior to 13Gy X-ray exposure (whole body).
5. Untreated, un-irradiated controls.

Intestinal damage was induced using a single dose of 13Gy X-irradiation. Four days after irradiation the animals were sacrificed. The small intestine was removed and fixed in Carnoy's fixative prior to processing for histological analysis. 3μm sections were cut and stained with haematoxylin and eosin. Foci of regeneration (surviving crypts with one or more clonogenic cells) were clearly visible in the irradiated sections. Other than these foci the mesenchyme was entirely denuded; these animals would develop diarrhoea and die due to mucositis if allowed to live beyond four days.

For each animal ten intestinal circumferences were analysed (60 per group) - a circumference is equivalent to a given length of intestine and therefore a convenient baseline unit of length. The number of surviving crypts per circumference was scored and the average per group determined. Only crypts containing 10 or more strongly haematoxylin and eosin stained cells (excluding Paneth cells) and only intact circumferences not containing Peyers patches were scored (Peyers patches influence both the number of crypts in a normal circumference and the ability of a crypt to survive insult).

The average crypt width (measured at its widest point) was also measured in order to correct for scoring errors due to crypt size difference. The correction is applied thus:

$$\text{Corrected number of crypts / circumference} =$$

$$\frac{\textit{Mean crypt width in untreated control}}{\textit{Mean crypt width in treated animal}} \times \textit{Mean number of surviving crypts in treatment group}$$

Compound 1.1 was given at 5, 10 and 20mg/kg daily for three days prior to radiation exposure. In animals treated with the vehicle 3.5 +/- 1.9 crypts per circumference (cross section) survived the insult. In each Compound 1.1 treated group the levels of survival were increased. Maximal survival was achieved at the highest dose (20mg/kg) where 6.7 +/- 3.0 crypts survived (1.9x increase). The lower doses increased survival about 1.3 times. These levels of protection can allow animal survival following an otherwise lethal dose of irradiation (assuming bone marrow damage is minimised) (Both reviewed in Booth & Potten 2001, JNCI Monogr, 29; 16-20).

All the animals survived the treatment and exhibited no obvious adverse effects.

The data are illustrated in Table 3 and Figure 3.

Pharmaceutical compositions

The route of administration is governed by the physical properties of the compound used and the type of mucositis to be treated and/or prevented. As discussed above, for mucositis treatment and/or prevention the compounds of formula (I) can be administered as oral formulations such as tablets, capsules, pills, or as syrups, oral rinse, gels and emulsions. Since the composition of the invention can be used also for preventing mucositis, administration of the compositions should preferably precede the initial dose of antineoplastic therapy or the radiation therapy by at least 24 hours.

The particular dosage of compounds of formula (I) required to prevent or treat mucositis or its symptoms,

according to this invention, will depend upon the severity of the condition, the route of administration and the related factors that will be decided by the attending physician. Generally, accepted and effective oral daily doses will be from about 0.5 to 500 mg/day (and more typically from about 10 to 100 mg/day). Such dosages will be administered to a subject in need thereof from once to about three times each day, or more often as needed, and for a sufficient duration, to effectively inhibit mucositis.

Suitable pharmaceutical compositions of compounds of formula (I) can be prepared by procedures known in the art. For example the compounds can be formulated with common excipients, diluents or carriers and formed into tablets, capsules, pills, mouth washes, suspensions or gels.

Examples of excipients, diluents, and carriers that are suitable for such formulations include but not limit to: fillers and extenders such as starch, lactose, mannitol, and silica derivatives; binding agents such as carboxymethyl cellulose and other cellulose derivatives, alginates, polyvinyl pyrrolidone.

Disintegrating agents such as calcium carbonate or sodium bicarbonate can be added where required. Lubricants such as talc, calcium and magnesium stearate or solid polyethyl glycols can be used for these compositions manufacturing, depending upon the physical properties of the compound of formula (I) to be formulated.

The compounds of the invention can also be formulated as suspensions or solutions for convenient oral administration or as solutions appropriate for parenteral administration, for instance by intramuscular, subcutaneous, or intravenous routes. The compositions of the invention can be in the form of a slightly viscous aqueous liquid (gel), which provides a film-forming and coating effect on the epithelial surfaces such as, but not limited to the oral mucosa.

## Claims

1. The use of a compound of formula (I), or its pharmaceutically acceptable salt or solvate, for preparing a medicament for treating or preventing mucositis induced by cancer therapy comprising chemotherapy and/or radiotherapy:

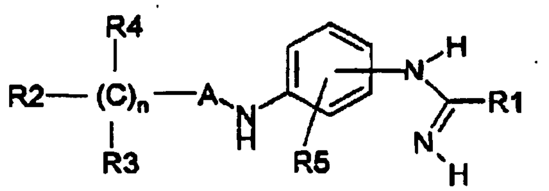

(I)

wherein:

- A is the thiocarboxamide group
- $R_1$ is selected from an alkyl group having from 1 to 3 carbon atoms and the amino group, unsubstituted or substituted with the nitro group or the methyl group,
- $R_2$ is selected independently from hydrogen, an alkyl group having from 1 to 4 carbon atoms, a cycloalkane residue having from 5 to 7 carbon atoms, an aryl, naphtyl or heterocyclic group, unsubstituted or substituted with methyl, methoxy, hydroxy, amino or halogen groups,
- $R_3$ and $R_4$ are selected independently from hydrogen and an alkyl group having from 1 to 3 carbon atoms,
- $R_5$ represents one or two substituents independently selected from hydrogen and the methyl, methoxy and hydroxyl groups,
- n is a whole number from 0 to 6, and
- the amidine group is in the *para* or *meta* position relative to the "A-NH" group.

2. The use according to claim 1, wherein in the compounds of formula (I) $R_1$ and $R_2$ are methyl groups, $R_3$ and $R_4$ and $R_5$ are hydrogen, n is a whole number from 0 to 6, and the amidine group is in the *para* position relative to the "A-NH" group, or a pharmaceutically acceptable salt or solvate thereof.

3. The use according to claim 1, wherein in the compounds of formula (I) $R_1$ and $R_2$ are methyl groups, $R_3$ and $R_4$ and $R_5$ are hydrogen, n is 4, and the amidine group is in the *para* position relative to the "A-NH" group, or a pharmaceutically acceptable salt or solvate thereof.

4. A compound as defined in any of claims 1 to 3, for use in the therapeutical treatment or prevention of mucositis

induced by cancer therapy, comprising chemotherapy and/or radiotherapy.

**5.** A pharmaceutical composition comprising a compound as defined in any of claims 1 to 3 and pharmaceutical excipients for use in the therapeutical treatment or prevention of mucositis induced by cancer therapy, comprising chemotherapy and/or radiotherapy.

**6.** A pharmaceutical composition for use according to claim 5, wherein said compound is a pharmaceutically acceptable salt of a compound of formula (I) selected from the group consisting of hydrochloride, hydrobromide, hydrogen sulphate, methansulphonate, maleate, fumarate and succinate.

**Patentansprüche**

**1.** Verwendung einer Verbindung der Formel (I), oder eines pharmazeutisch unbedenklichen Salzes oder Solvats derselben, zur Herstellung eines Arzneimittels zur Behandlung oder Verhütung einer Schleimhautentzündung, die durch Krebstherapie, einschließlich Chemotherapie und/oder Strahlentherapie, hervorgerufen wird:

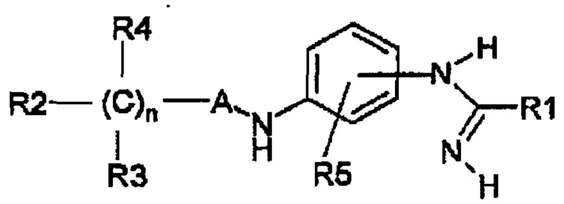

(I)

wobei:

- A die Carbothioamidgruppe ist,
- $R_1$ aus einer Alkylgruppe, die über 1 bis 3 Kohlenstoffatome und die Aminogruppe verfügt, unsubstituiert oder mit der Nitrogruppe oder der Methylgruppe substituiert, ausgewählt ist,
- $R_2$ unabhängig davon aus Wasserstoff, einer Alkylgruppe mit 1 bis 4 Kohlenstoffatomen, einem Cycloalkanrest mit 5 bis 7 Kohlenstoffatomen, einer Aryl-, Naphthyl- oder heterozyklischen Gruppe, unsubstituiert oder mit Methyl-, Methoxy-, Hydroxy-, Amino- oder Halogengruppen substituiert, ausgewählt ist,
- $R^3$ und $R^4$ unabhängig voneinander aus Wasserstoff und einer Alkylgruppe mit 1 bis 3 Kohlenstoffatomen ausgewählt sind,
- $R^5$ für einen oder zwei Substituenten steht, die unabhängig voneinander aus Wasserstoff und den Gruppen Methyl, Methoxy und Hydroxyl ausgewählt sind,
- n eine ganze Zahl von 0 bis 6 ist, und
- die Amidingruppe sich in der *para*- oder meta-Stellung zur "A-NH"-Gruppe befindet.

**2.** Verwendung gemäß Anspruch 1, wobei in den Verbindungen der Formel (I) $R_1$ und $R_2$ Methylgruppen sind, $R_3$ und $R_4$ und $R_5$ Wasserstoff sind, n eine ganze Zahl von 0 bis 6 ist, und die Amidingruppe sich in *para*-Stellung zur "A-NH"-Gruppe befindet, oder ein pharmazeutisch unbedenkliches Salz oder Solvat davon.

**3.** Verwendung gemäß Anspruch 1, wobei in den Verbindungen der Formel (I) $R_1$ und $R_2$ Methylgruppen sind, $R_3$ und $R_4$ und $R_5$ Wasserstoff sind, n gleich 4 ist, und die Amidingruppe sich in *para*-Stellung zur "A-NH"-Gruppe befindet, oder ein pharmazeutisch unbedenkliches Salz oder Solvat davon.

**4.** Verbindung wie in einem der Ansprüche 1 bis 3 festgelegt, zur Verwendung bei der therapeutischen Behandlung oder der Verhütung einer Schleimhautentzündung, die durch Krebstherapie, einschließlich Chemotherapie und/oder Strahlentherapie, hervorgerufen wird.

**5.** Pharmazeutische Zusammensetzung umfassend eine Verbindung wie in einem der Ansprüche 1 bis 3 festgelegt, sowie pharmazeutische Trägerstoffe zur Verwendung bei der therapeutischen Behandlung oder der Verhütung einer Schleimhautentzündungen, die durch Krebstherapie, einschließlich Chemotherapie und/oder Strahlentherapie, hervorgerufen wird.

**6.** Pharmazeutische Zusammensetzung für eine Verwendung gemäß Anspruch 5, wobei die Verbindung ein pharmazeutisch unbedenkliches Salz einer Verbindung der Formel (I) ist, welches aus der Gruppe bestehend aus Hydrochlorid, Hydrobromid, Hydrogensulfat, Methansulfonat, Maleat, Fumarat und Succinat ausgewählt ist.

## Revendications

**1.** Utilisation d'un composé de formule (I), ou de son sel ou solvate pharmaceutiquement acceptable, pour préparer un médicament destiné à traiter ou prévenir une inflammation des muqueuses induite par un traitement anticancéreux comprenant chimiothérapie et/ou radiothérapie :

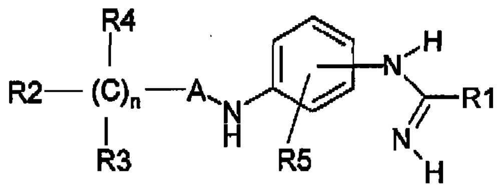

(I)

où :

- A est le groupe thiocarboxamide,
- $R_1$ est sélectionné parmi un groupe alkyle ayant de 1 à 3 atomes de carbone et le groupe amino, non substitué ou substitué par le groupe nitro ou le groupe méthyle,
- $R_2$ est sélectionné indépendamment parmi l'hydrogène, un groupe alkyle ayant de 1 à 4 atomes de carbone, un résidu cycloalkane ayant de 5 à 7 atomes de carbone, un groupe aryle, naphtyle ou hétérocyclique, non substitué ou substitué par des groupes méthyle, méthoxy, hydroxy, amino ou halogène.
- $R_3$ et $R_4$ sont sélectionnés indépendamment parmi l'hydrogène et un groupe alkyle ayant de 1 à 3 atomes de carbone,
- $R_5$ représente un ou deux substituants sélectionnés indépendamment parmi l'hydrogène et les groupes méthyle, méthoxy et hydroxyle,
- n est un entier de 0 à 6, et
- le groupe amidine est à la position *para* ou *méta* par rapport au groupe "A-NH".

**2.** Utilisation selon la revendication 1, où dans les composés de formule (I) $R_1$ et $R_2$ sont des groupes méthyle, $R_3$ et $R_4$ et $R_5$ sont l'hydrogène, n est un entier de 0 à 6, et le groupe amidine est à la position *para* par rapport au groupe "A-NH", ou sel ou solvate pharmaceutiquement acceptables de ce dernier.

**3.** Utilisation selon la revendication 1, où dans les composés de formule (I) $R_1$ et $R_2$ sont des groupes méthyle, $R_3$ et $R_4$ et $R_5$ sont l'hydrogène, n est égal à 4 et le groupe amidine est à la position *para* par rapport au groupe "A-NH", ou sel ou solvate pharmaceutiquement acceptable de ce dernier.

**4.** Composé selon l'une quelconque des revendications 1 à 3, pour utilisation dans le traitement thérapeutique ou la prévention d'une inflammation des muqueuses induite par un traitement anticancéreux comprenant chimiothérapie et/ou radiothérapie.

**5.** Composition pharmaceutique comprenant un composé selon l'une quelconque des revendications 1 à 3 et des excipients pharmaceutiques pour utilisation dans le traitement thérapeutique ou la prévention d'une inflammation des muqueuses induite par un traitement anticancéreux comprenant chimiothérapie et/ou radiothérapie.

**6.** Composition pharmaceutique pour utilisation selon la revendication 5, où ledit composant est un sel pharmaceutiquement acceptable d'un composé de formule (I) sélectionné dans le groupe constitué de chlorhydrate, bromhydrate, hydrogénosulfate, méthanesulfate, maléate, fumarate et succinate.

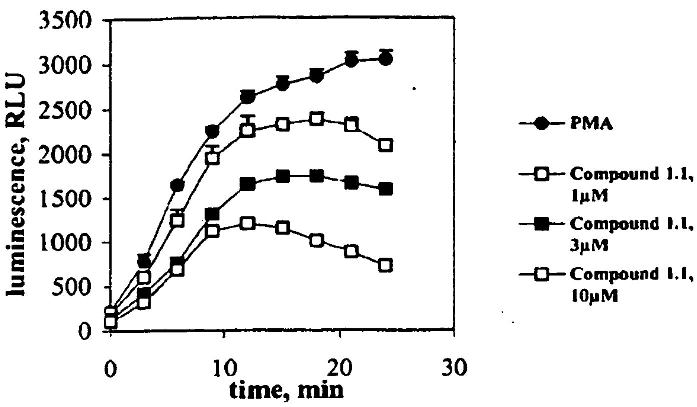


**Figure 1 :** Effect of Compound 1.1 (1-10 μM) on chemiluminescence induced by 0.1μM Phorbol 12-Myristate 13-Acetate (PMA) in h-PMNL. Pre-treatment with Compound 1.1 diminished the response to PMA in a concentration-dependent manner as compared to the effect of Compound 1.1 vehicle.

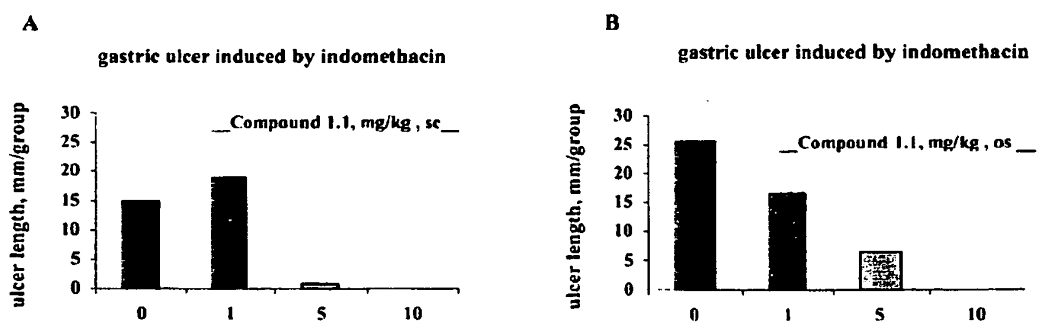


**Figure 2:** Compound 1.1 (1-10 mg/kg), dose-dependently reduced the formation of gastric ulcers induced by indomethacin. The effect was achieved both after systemic administration (*sc* route, 15 min prior to indomethacin), panel A, and after oral administration (*os* route, 30 min prior to indomethacin), panel B.

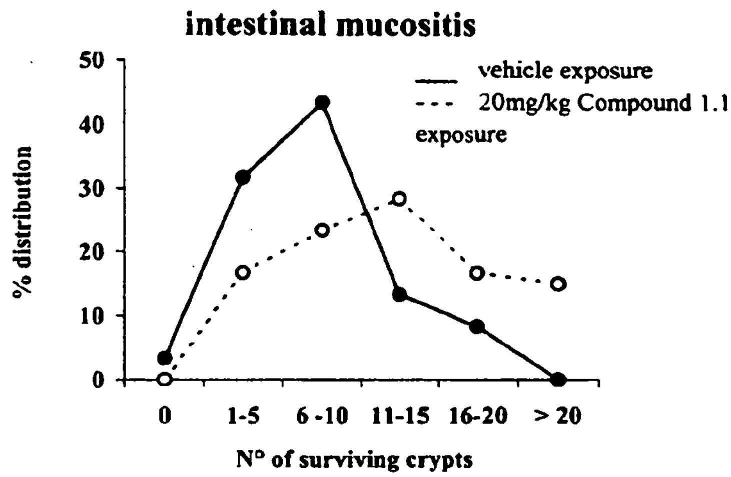


**Figure 3:** Compound 1.1 reduced the intestinal crypt death induced by 13Gy irradiation in mouse.

Compound 1.1 at 20mg/kg prevented the absence of surviving crypt (as seen in 4% of circumference in vehicle treated mouse), and increased the percentage of surviving crypts per circumference.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9945910 A
- WO 02070468 A


### Non-patent literature cited in the description


- **M. DUNCAN ; GRANT G.** *Aliment. Pharmacol. Ther.,* 2003, vol. 18 (9), 853-74
- **SONIS ST et al.** *Cancer,* 2004, vol. 9 (100), 1995-2025
- **FACORRO G et al.** *Bone Marrow Transplant.,* 2004, vol. 33 (8), 793-8
- **SONIS ST et al.** *Cell Prolif.,* 2002, vol. 35 (1), 93-102
- **SONIS ST.** *Nat rev Cancer,* 2004, vol. 4 (4), 277-284
- **SONIS ST et al.** *Oral Oncol.,* 2004, vol. 40 (2), 170-6
- **Rubenstein EB et al.** *Cancer suppl.,* 2004, vol. 100 (9), 2026-2046
- **Worthington HV et al.** *Cochrane Review,* 2004, 3
- **BOYUM A.** *Scand J Clin Invest,* 1968, vol. 21, 77-89
- *Methods in Enzymology,* vol. LVIII, 494-506
- **BOOTH ; POTTEN.** *JNCI Monogr,* 2001, vol. 29, 16-20